(19)

Europäisches
Patentamt
European
Patent Office
Office européen
des brevets

(11)  **EP 4 527 288 A1**

(12)  **EUROPEAN PATENT APPLICATION**
published in accordance with Art. 153(4) EPC

(43) Date of publication:
26.03.2025 Bulletin 2025/13

(21) Application number: 23807901.6

(22) Date of filing: 17.05.2023

(51) International Patent Classification (IPC):
*A61B 5/00* (2006.01)    *A61B 5/021* (2006.01)
*A61B 5/053* (2021.01)    *A61B 5/024* (2006.01)
*A61B 5/145* (2006.01)

(52) Cooperative Patent Classification (CPC):
A61B 5/00; A61B 5/021; A61B 5/024; A61B 5/053;
A61B 5/145

(86) International application number:
PCT/KR2023/006669

(87) International publication number:
WO 2023/224382 (23.11.2023 Gazette 2023/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 18.05.2022 KR 20220060695

(71) Applicant: **Park, Eul Joon**
**Seoul 06356 (KR)**

(72) Inventor: **PARK, Eul Joon**
**Seoul 06356 (KR)**

(74) Representative: **WP Thompson**
**138 Fetter Lane**
**London EC4A 1BT (GB)**

(54)  **SUBJECT PREDICTION METHOD AND DEVICE FOR SYMPATHECTOMY**

(57)  A method for predicting a subject for sympathectomy includes: collecting first response data before a perturbation during an autonomic nerve assessment test; collecting second response data after the perturbation during the autonomic nerve assessment test; and judging whether a sympathectomy is required using a first difference value between the first response data and the second response data. The autonomic nerve assessment test includes at least one of a head-up tilt (HUT) test, a Valsalva maneuver, and a cold pressure test.

FIG. 1

**Description**

FIELD OF THE DISCLOSURE

Embodiments relate to a method and device for predicting a subject for sympathectomy. More specifically, embodiments relate to a device and method for predicting a subject for sympathectomy that predict or select appropriate subjects (patients) that will respond to sympathectomy.

BACKGROUND OF THE INVENTION

Abnormally high sympathetic nerve activation exists in patients with chronic diseases such as hypertension, kidney disease, heart failure, diabetes, etc. Sympathetic modulation, such as renal sympathetic depression, is used to treat these chronic conditions.

In addition, various treatments for patients with the above-mentioned chronic diseases are being studied. However, in identifying patients with the above-mentioned diseases, a clinically applicable and non-invasive method for such identification is not clearly presented.

Accordingly, the present invention relates to a clinically applicable and non-invasive method for identifying patients with the above-mentioned diseases, and provides a very important prediction or identification method in the treatment of patients whose disease needs to be treated by lowering sympathetic nerve activity.

The present invention according to embodiments may provide a method and device for predicting a subject for sympathectomy that may predict or identify patients with chronic diseases (e.g., hypertension, heart failure, atrial fibrillation, sleep apnea, diabetes, and renal failure) that result in abnormally elevated nervous (e.g., sympathetic).

Specifically, the present invention may provide a method and device for predicting a subject for sympathectomy that provides parameters that may provide absolute values, relative changes, and/or reserves (amounts of change) in sympathetic nerve activity.

In addition, the above-mentioned parameters are provided from bioelectrical, hemodynamic signals and/or neurohormonal responses, and the level of the sympathetic nerve in the patient may be disturbed in a non -invasive or invasive way, any of the head-up tilt (HUT), the Valsalva Maneuver (V.M) and the cold pressure test (C.P).

In addition, according to an embodiment, a method and device for predicting a subject for sympathectomy that may more accurately perform the distinguishment for patients with abnormally high sympathetic nerve activation may be provided.

The objects to be achieved in the embodiment are not limited thereto, and it can be said that the objects and effects that may be understood from the means of solving the problems and embodiments described below are also included.

A method for predicting a subject for sympathectomy according to an embodiment comprises the steps of collecting first response data before a perturbation during an autonomic nerve assessment test; collecting second response data after the perturbation during the autonomic nerve assessment test; and judging whether a sympathectomy is required, by using a first difference value between the first response data and the second response data, wherein the autonomic nerve assessment test is any one of head-up tilt (HUT) test, Valsalva maneuver, and cold pressure test.

The method comprises the step of collecting third response data before the perturbation during the autonomic nerve assessment test, and the third response data may be the first response data after administration of a sympatholytic drug.

If a ratio of the first difference value to a second difference value is within a predetermined range, it may be judged that the sympathectomy or sympathetic denervation is required, and the second difference value may be a difference value between the second response data and the third response data.

The second difference value may be greater than the first difference value, and the second difference value may be a difference value between the second response data and the third response data.

The sympatholytic drug may include any one of dopamine, dobutamine, adenosine, prostacyclin, nitric oxide, bradykinin, papaverine, dipyridamolum, diuretin, theophylline, minoxidil and isosorbide may be included.

In the judgment step, it is judged that the sympathectomy or sympathetic denervation is required when a ratio of the first difference value to an average difference is within a predetermined range.

The first response data or the second response data may be any one of central blood pressure, electrical resistance (bio impedance), heart rate variability (HRV), amount of epinephrine/norepinephrine in blood and skin sympathetic nerve activity (SSNA).

When the first response data or the second response data is any one of the central blood pressure, amount of epinephrine/norepinephrine, and the skin sympathetic nerve activity, the first response data may be lower than the second response data.

When the first response data or the second response data is the electrical resistance, the first response data may be greater than the second response data.

When the first response data or the second response data is the heart rate variability, the first response data may be greater than the second response data in a low frequency region.

In a non-transitory computer-readable storage medium on which a program including at least one instruction for performing a method for predicting a subject for sympathectomy is recorded according to an embodiment, the method for predicting a subject for sympathectomy comprises the steps of collecting first response data before a perturbation during an autonomic nerve assessment test; collecting second response data after the perturbation during the autonomic nerve assessment test; and judging whether a sympathectomy is required, by using a first difference value between the first response data and the second response data, wherein the autonomic nerve assessment test is any one of head-up tilt (HUT) test, Valsalva maneuver, and cold pressure test.

A device for predicting a subject for sympathectomy according to an embodiment comprises a processor performing at least one instruction, wherein the processor is configured to collect first response data before a perturbation during an autonomic nerve assessment test, collect second response data after the perturbation during the autonomic nerve assessment test, and judge whether a sympathectomy is required, by using a first difference value between the first response data and the second response data, wherein the autonomic nerve assessment test is any one of head-up tilt (HUT) test, Valsalva maneuver, and cold pressure test.

The processor may be configured to collect third response data before the perturbation during the autonomic nerve assessment test, and the third response data may be the first response data after administration of a sympatholytic drug.

The processor may be configured to judge that the sympathectomy or sympathetic denervation is required if a ratio of the first difference value to a second difference value is within a predetermined range, and the second difference value may be a difference value between the second response data and the third response data.

The second difference value may be greater than the first difference value, and the second difference value may be a difference value between the second response data and the third response data.

According to an embodiment, the present invention provides a method for predicting or identifying patients with chronic diseases (e.g., hypertension, heart failure, atrial fibrillation, sleep apnea, diabetes, renal failure, etc.) in which nerve (e.g., sympathetic) activity is abnormally elevated.

Specifically, the present invention may implement a method and device for predicting a subject for sympathectomy that provide parameters that may provide absolute values, relative changes and/or reserves (reservoirs or changeable amounts) in sympathetic nerve activity.

In addition, the present invention may implement a method and device for predicting a subject for sympathectomy that may predict a subject for sympathectomy using a non-invasive or invasive method.

In addition, according to an embodiment, a method and device for predicting a subject for sympathectomy that may more accurately perform the distinguishment for patients with abnormally high sympathetic nerve activation may be implemented.

The various and beneficial advantages and effects of the present invention are not limited to the above-described content, and may be more easily understood through description of specific embodiments of the present invention.

BRIEF DESCRIPTION OF THE DRAWINGS

FIG. 1 is a block diagram schematically illustrating a device for predicting a subject for sympathectomy according to an embodiment of the present invention.

FIG. 2A is a flowchart of a method for predicting a subject for sympathectomy according to an embodiment of the present invention.

FIG. 2B is a diagram explaining a result of an autonomic nerve assessment test between a patient and the general public in a method for predicting a subject for sympathectomy according to an embodiment of the present invention.

FIG. 3 is a detailed flowchart of a method for predicting a subject for sympathectomy according to an embodiment of the present invention.

FIG. 4 is a diagram explaining an effect of a method for predicting a subject for sympathectomy according to an embodiment of the present invention.

FIG. 5 is a detailed flowchart of a method for predicting a subject for sympathectomy according to another embodiment of the present invention.

FIG. 6 is a block diagram illustrating an example of a method and device for predicting a subject for sympathectomy according to an embodiment of the present invention.

FIGS. 7 to 12 are graphs illustrating any one result of central blood pressure, electrical resistance (bio impedance), heart rate variability (HRV), amount of epinephrine in the blood, and skin sympathetic nerve activity (SSNA) for any one of head-up tilt test (HUT), Valsalva maneuver, and cold pressure test in an embodiment.

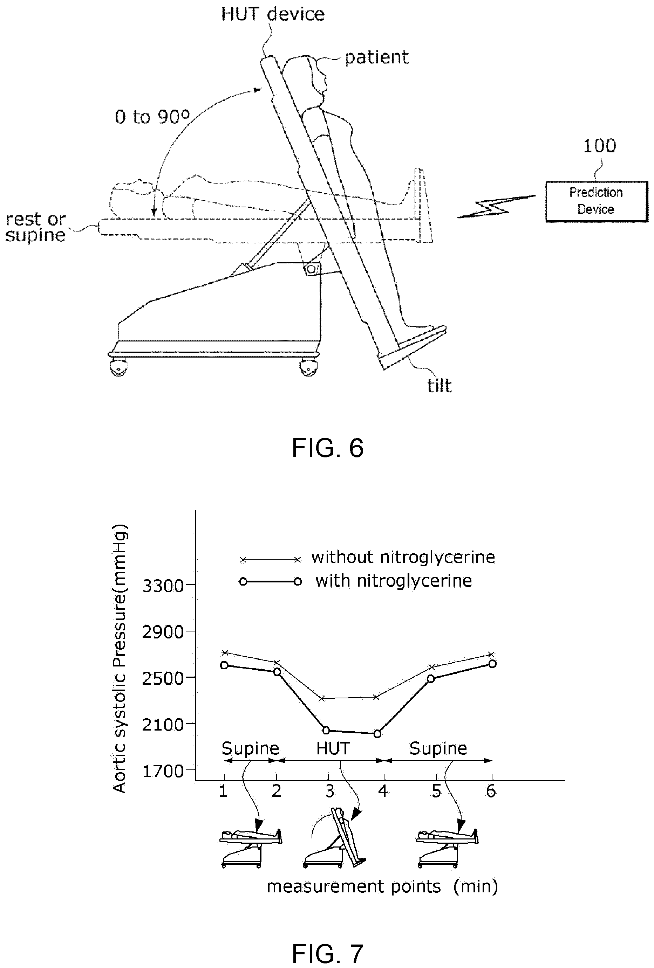

FIG. 7 is a graph of central blood pressure, such as aortic systolic pressure, for a head-up tilt test.

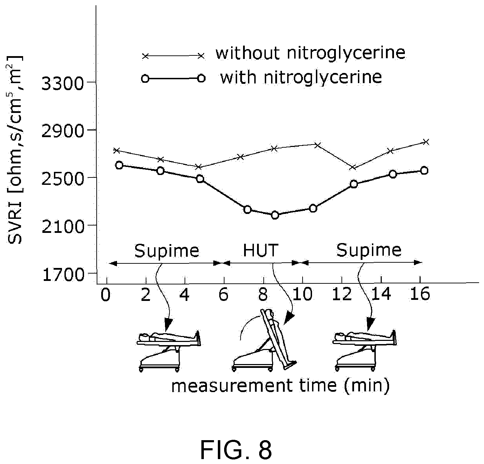

FIG. 8 is a graph of systemic vascular resistance index (SVRI) for a head-up tilt test.

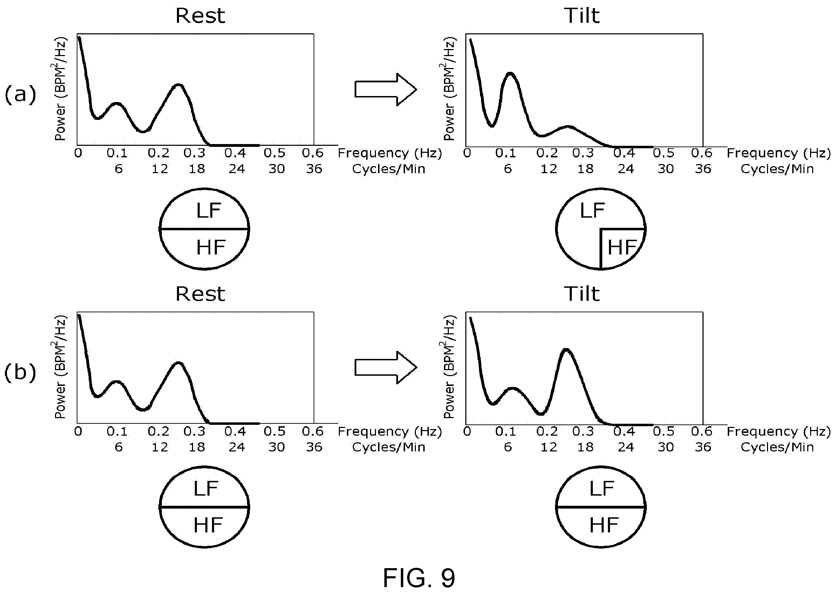

FIG. 9 is a graph of heart rate variability (power spectrum) for a head-up tilt test.

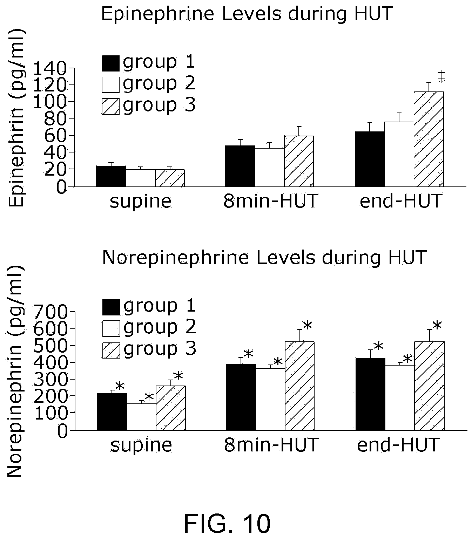

FIG. 10 is a graph of epinephrine and norepinephrine in the blood for a head-up tilt test.

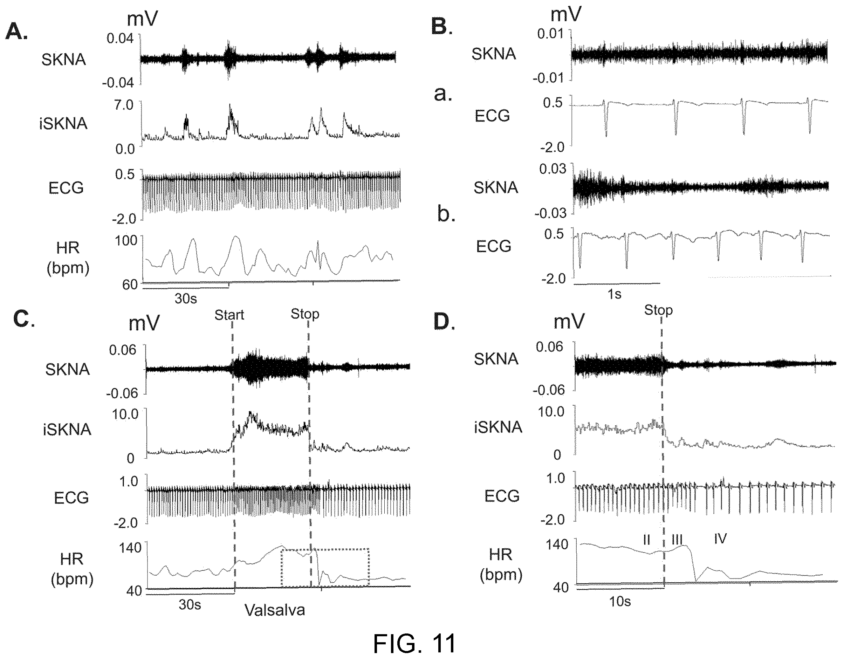

FIG. 11 is a graph of skin sympathetic nerve activity for Valsalva maneuver.

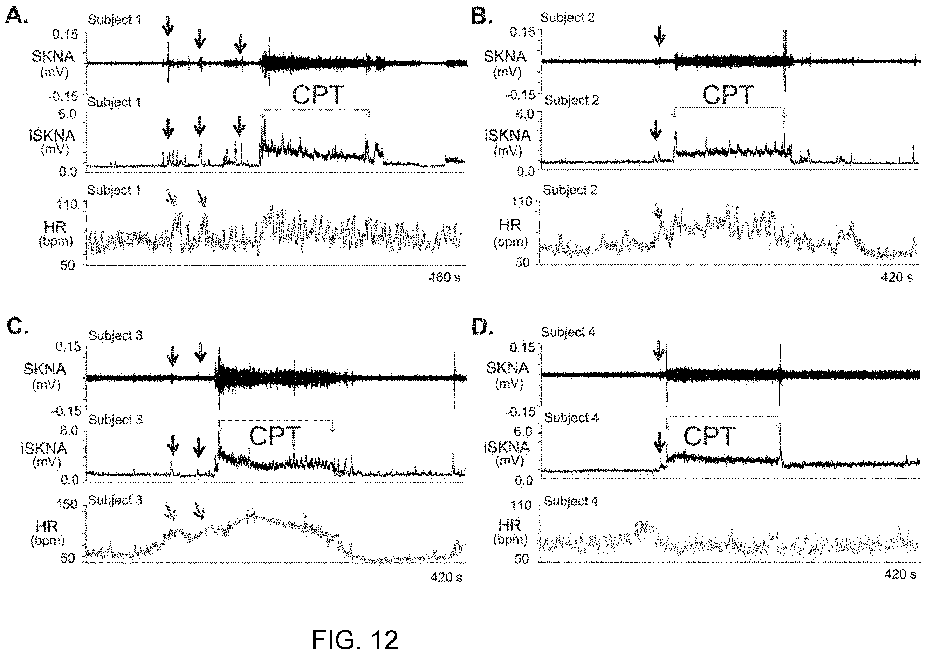

FIG. 12 is a graph of skin sympathetic nerve activity for a cold pressure test.

DETAILED DESCRIPTION OF THE INVENTION

The present invention may have various modifications and various exemplary embodiments and specific exemplary embodiments will be illustrated in the drawings and described. However, this does not limit the present invention to specific exemplary embodiments, and it should be understood that the present invention covers all the modifications, equivalents and replacements within the idea and technical scope of the present invention.

Terms including an ordinal number such as first or second may be used to describe various components but the components are not limited by the above terms. The above terms are used only to discriminate one component from the other component. For example, without departing from the scope of the present invention, a second component may be referred to as a first component, and similarly, the first component may be referred to as the second component. A terminology such as and/or includes a combination of a plurality of associated items or any item of the plurality of associated items.

It should be understood that, when it is described that an element is "coupled" or "connected" to another element, the element may be "directly coupled" or "directly connected" to the other element or "coupled" or "connected" to the other element through a third element. In contrast, it should be understood that, when it is described that an element is "directly coupled" or "directly connected" to another element, it is understood that no element is not present between the element and the other element.

Terms used in the present application are used only to describe specific exemplary embodiments, and are not intended to limit the present invention. A singular form may include a plural form if there is no clearly opposite meaning in the context. In the present application, it should be understood that term "include" or "have" indicates that a feature, a number, a step, an operation, a component, a part or the combination thereof described in the specification is present, but does not exclude a possibility of presence or addition of one or more other features, numbers, steps, operations, components, parts or combinations, in advance.

If it is not contrarily defined, all terms used herein including technological or scientific terms have the same meaning as those generally understood by a person with ordinary skill in the art. It should be understood that terms defined in a generally used dictionary have the same meanings as contextual meanings of associated techniques and if not apparently defined in this application, the terms should not be interpreted as ideological or excessively formal meaning.

In addition, some embodiments may be described in terms of functional block components and various processing steps. Some or all of such functional blocks may be realized by any number of hardware and/or software components configured to perform the specified functions. For example, functional blocks of the disclosure may be implemented by one or more microprocessors or by circuit components for performing intended functions. Also, for example, the functional blocks according to the disclosure may be implemented using various programming or scripting language. Functional blocks may be implemented in algorithms that execute on one or more processors. Furthermore, the disclosure could employ any number of techniques according to the related art for electronics configuration, signal processing and/or data processing, and the like. The words "modules" and "components" are used broadly and are not limited to mechanical or physical components.

Hereinafter, exemplary embodiments of the present invention will be described in detail with reference to the accompanying drawings, in which like reference numerals refer to like or similar elements regardless of reference numerals and a duplicated description thereof will be omitted.

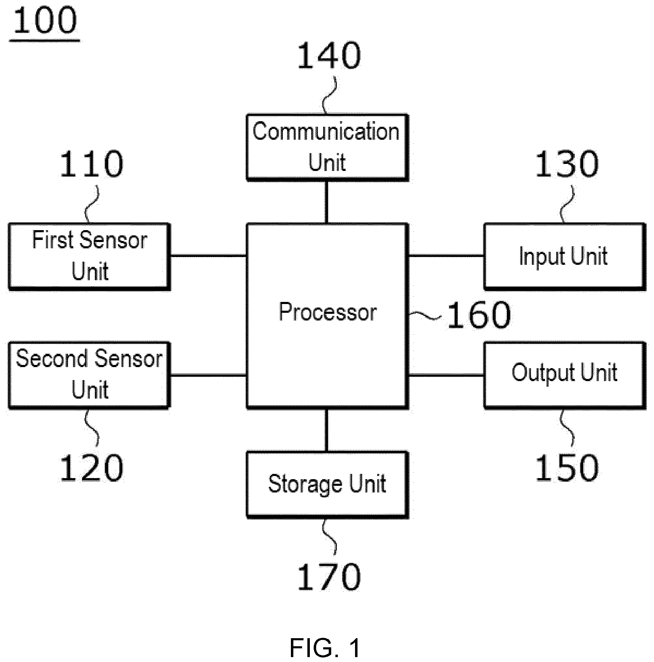

FIG. 1 is a block diagram schematically illustrating a device for predicting a subject for sympathectomy according to an embodiment of the present invention.

Referring to FIG. 1, a device for predicting a subject for sympathectomy 100 according to an embodiment of the present invention may comprise a first sensor unit 110, a second sensor unit 120, an input unit 130, a communication unit 140, and a storage unit 150, a processor 160, and an output unit 170.

The first sensor unit 110 is configured to include a plurality of sensors and may include a sensor that detects the patient's condition for the test or examination. The test or examination to be performed for a subject (hereinafter used interchangeably with 'patient') may be any of the head-up tilt test (HUT), Valsalva maneuver, and cold pressure test.

For example, the first sensor unit 110 may include a sensor that measures the patient's head-up tilt and a sensor that measures the patient's movement. Accordingly, the first sensor unit 110 may include a gyro sensor and an acceleration sensor.

The second sensor unit 120 is configured to include one or more sensors and may further include a sensor that measures the patient's biological condition. For example, the second sensor unit 120 may include a sensor that detects the patient's condition for the test or examination.

The second sensor unit 120 may include one or more of a vibration sensor, an acoustic sensor, a pulse measurement sensor, an electrocardiogram sensor, a blood pressure sensor, a respiration measurement sensor, an EEG measurement sensor, a cerebral blood flow sensor, an impedance measurement sensor, a hormone detection sensor, and a skin sensation detection sensor. In an embodiment, the second sensor unit may include a sensor indicating any one of central blood pressure, electrical resistance (bio impedance), heart rate variability (HRV), amount of epinephrine in the blood, and skin sympathetic nerve activity (SSNA) in the patient's biological condition.

The input unit 130 converts the user's input operation into an input signal and transmits the input signal to the processor 160. The input unit 130 may be implemented, for example, as a keyboard, mouse, touch sensor on a touch screen, touchpad, keypad, voice input, and other input processing devices that are currently available in the past or will be available in the future. For example, the input unit 130 may receive personal settings, including the user's daily training angle and training time, and store them in the storage unit 150 .

The communication unit 140 transmits and receives information to and from external terminals and servers. For example, the communication unit 140 may receive settings for prediction judgment according to an embodiment from an external terminal and server and store them in the storage unit 150. In addition, the communication technology used by the communication unit 140 may vary depending on the type of communication network or other circumstances.

The storage unit 150 stores programs and data to provide users with services for testing or exams (e.g., self-head-up tilt test). In addition, the storage unit 150 may store data about a biosignal for a test or examination. In addition, the storage unit 150 stores information generated during a test or examination, and may transmit the requested data to the processor 160 upon request from the processor 160. The storage unit 150 may include read-only memory (ROM), random access memory (RAM), flash memory, a memory card, a storage medium, and/or other storage devices.

The processor 160 may perform each step of the method for predicting a subject for sympathectomy according to an embodiment described later. Accordingly, the device for predicting a subject for sympathectomy according to an embodiment 100 may perform the method for predicting a subject for sympathectomy through the processor 160. In addition, for example, an electronic device (including a device for predicting a subject for sympathectomy) may include the above-described processor, and the processor may perform a method for predicting a subject for sympathectomy to be described later.

Furthermore, this device for predicting a subject for sympathectomy may be within or connected to each device for autonomic nerve assessment. Alternatively, it may be programmed and performed within each piece of equipment that performs an autonomic nerve assessment test, or may be programmed and performed within a device connected to each piece of equipment.

This processor 160 may include an application-specific integrated circuit (ASIC), other chipset, logic circuit, and/or data processing device.

The output unit 170 may output a message according to an indication or instruction of the processor 160 in one or more of visual, tactile, and auditory ways. The output unit 170 may include one or more of a display and a speaker. The display may be implemented as, for example, a liquid crystal display (LCD), a light emitting diode (LED), an organic light emitting diode (OLED), a projector, or other display devices that are currently available, have been available in the past, or will be available in the future.

In an embodiment of the present invention, it has been described that the device for predicting a subject for sympathectomy 100 is an integral component of the first and second sensor units. However, the first and second sensor units may be individual components, and in this case, the first and second sensor units are implemented to transmit and receive information with the device for predicting a subject for sympathectomy 100. For example, the device for predicting a subject for sympathectomy 100 may be an electronic device, and the first and second sensor units may be various wearable devices. For example, in some cases, a self-head-up tilt test may be performed through a wearable type device.

In addition, when the device for predicting a subject for sympathectomy 100 transmits and receives data, depending on the viewpoint, the communication unit 140 may transmit and receive data under the control of the processor 160, and the processor 160 may control the communication unit 140 to transmit and receive data.

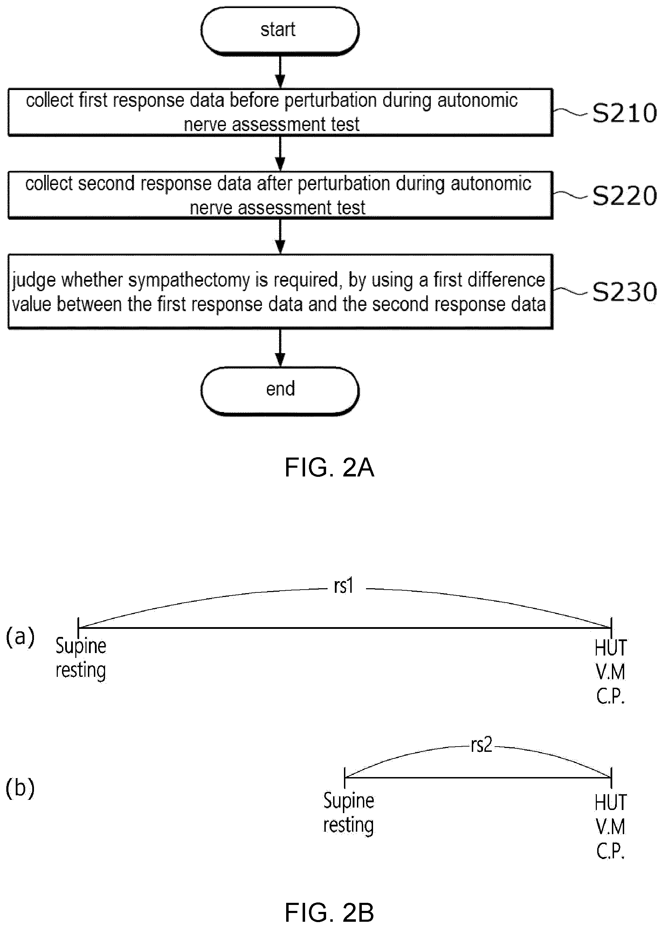

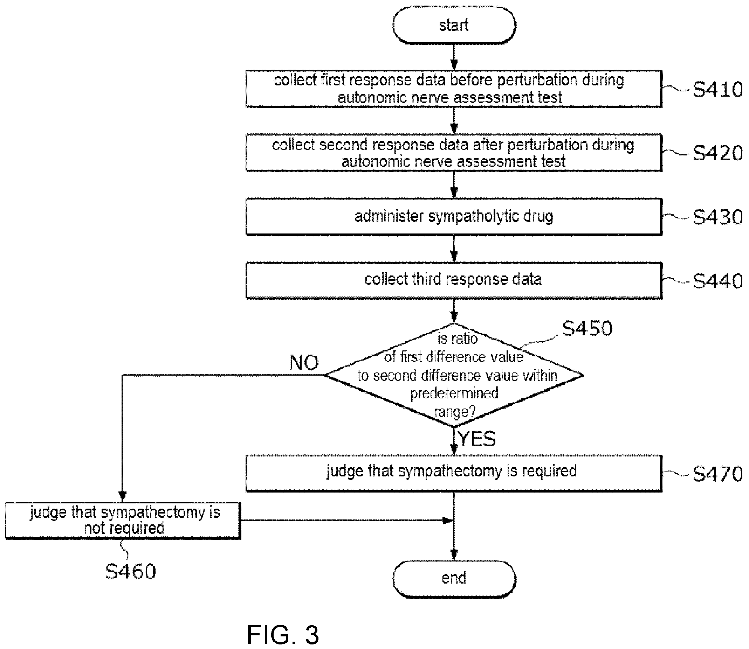

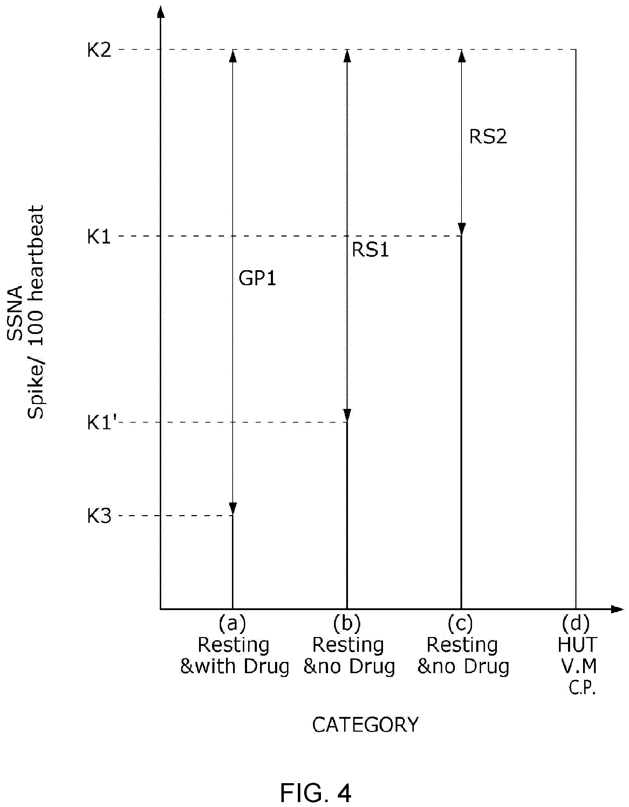

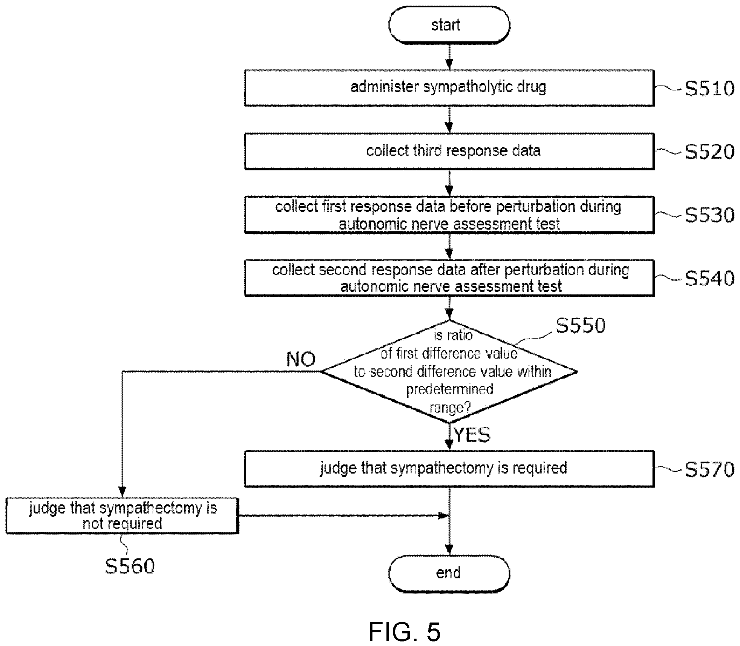

FIG. 2A is a flowchart of a method for predicting a subject for sympathectomy according to an embodiment of the present invention, FIG. 2B is a diagram explaining a result of an autonomic nerve assessment test between a patient and the general public in a method for predicting a subject for sympathectomy according to an embodiment of the present invention, FIG. 3 is a detailed flowchart of a method for predicting a subject for sympathectomy according to an embodiment of the present invention, FIG. 4 is a diagram explaining an effect of a method for predicting a subject for sympathectomy according to an embodiment of the present invention, FIG. 5 is a detailed flowchart of a method for predicting a subject for sympathectomy according to another embodiment of the present invention.

Referring to FIG. 2A, a method for predicting a subject for sympathectomy according to an embodiment of the present invention may comprise the steps of collecting first response data before a perturbation during an autonomic nerve assessment test (S210); collecting second response data after the perturbation during the autonomic nerve assessment test (S220); and judging whether a sympathectomy is required, by using a first difference value between the first response data and the second response data (230).

The autonomic nerve assessment test may be any one of head-up tilt (HUT) test, Valsalva maneuver (V.M), and cold pressure test (C.P). That is, in the autonomic nerve assessment test, the test state may be detected by the first sensor unit. Also, 'before perturbation during autonomic nerve assessment test' may correspond to a resting state before changes in the autonomic nerves. For example, in the head-up tilt test, 'before perturbation during the autonomic nerve assessment test' is before tilt, which may correspond to a supine or resting state. In addition, in the Valsalva maneuver (V.M.), 'before perturbation during the autonomic nerve assessment test' may correspond to a state before inducing a deep breath and holding the breath. In addition, in the cold pressure test, 'before perturbation during the autonomic nerve assessment test' may responded to a state before immersing the hand in cold water.

Furthermore, the first response data and second response data may be data measuring the patient's biological condition. For example, in this specification, response data may be data resulting from detecting the biological state of a subject (patient) by the second sensor unit described above. In other words, the response data may be data representing any one of central blood pressure, electrical resistance (bio impedance), heart rate variability (HRV), amount of epinephrine (or norepinephrine) in the blood, and skin sympathetic nerve activity (SSNA). For example, response data may include central blood pressure, the subject's electrical resistance, etc.

Accordingly, the device or processor for predicting a subject for sympathectomy according to an embodiment may collect first response data before perturbation during the autonomic nerve assessment test (S210). In other words, response data may be collected in the supine or rest state in the head-up tilt test (HUT). Furthermore, in the following, each step in addition to the injection of the sympatholytic drug will be explained based on assumption that each step is performed by the prediction device or processor. Further, in the present specification, any one of dopamine, dobutamine, adenosine, prostacyclin, nitric oxide, bradykinin, papaverine, dipyridamolum, diuretin, theophylline, minoxidil and isosorbide may be included.

In addition, the second response data after perturbation during the autonomic nerve assessment test may be collected (S220). As described above, the second response data may be a result of detecting a biological state after the examination or test. In other words, 'after perturbation during the autonomic nerve assessment test' may correspond to the state of change (noted as HUT, V.M, C.P) after autonomic nerve change. For example, in the head-up tilt test, 'after perturbation during the autonomic nerve assessment test' may correspond to the tilt state after the tilt. In addition, in the Valsalva maneuver (V.M.), 'after perturbation during the autonomic nerve assessment test' may correspond to a state after inducing a deep breath and holding the breath. In addition, in the cold pressure test, 'after perturbation during the autonomic nerve assessment test' may correspond to a state after immersing the hand in cold water.

Furthermore, it is possible to judge whether sympathectomy is required using a first difference value between the first response data and the second response data (S230). Subjects or patients who require sympathectomy or sympathetic denervation are those with abnormally high sympathetic nerve activation, and are patients for whom sympathectomy or sympathetic denervation may show a significant improvement in diseases such as hypertension. In contrast, in patients with normal sympathetic nerve activation, sympathectomy or sympathetic denervation does not have a significant improvement effect, so it can be judged that procedures such as sympathectomy or sympathetic denervation are not performed. Accordingly, it is possible to more accurately judge whether a patient is experiencing significant improvement due to sympathectomy sympathetic denervation.

Considering a size of a first difference value, which is a difference value between the first response data and the second response data, or a ratio with an average difference value (a first difference value) of a patient (e.g., a patient without hypertension) whose sympathetic nerve activation is not abnormally high, it may be judged whether sympathectomy or sympathetic denervation is required. Furthermore, for patients whose sympathetic nerve activation is not abnormally high, a subject to be measured, that is, the patient's age, may be applied so the patients may be compared by age group and it may be determined whether ablation is required as described above. In addition, through this, for patients who do not administer the sympatholytic drug, it may distinguish whether they are patients with abnormally high sympathetic nerve activation.

More specifically, referring to FIG. 2A, in the case of the general public (a), a difference value RS1 between a response data (first response data) 'before the perturbation during the autonomic nerve assessment test (supine)' and response data (second response data) 'after the perturbation during the autonomic nerve assessment test (HUT, V.M. C.P) may be larger than the difference value RS2 of patient (b). Here, the difference value RS 1 refers to the dose or changeable amount of the general public (or patients whose sympathetic nerve activation is not abnormally high). Also, the difference value RS2 refers to the dose or changeable amount in patients with abnormally high sympathetic nerve activation. In other words, the present invention may easily distinguish patients whose sympathetic nerve activation is not abnormally high from the general population.

In addition, the performance or function in each step described above may be equally applied to each embodiment described later.

Referring to FIG. 3, a method for predicting a subject for sympathectomy according to an embodiment of the present invention may comprise the steps of collecting first response data before a perturbation during an autonomic nerve assessment test (S410); collecting second response data after the perturbation during the autonomic nerve assessment test (S420); collecting third response data before the perturbation during the autonomic nerve assessment test (S440), and judging whether a sympathectomy is required, by using a first difference value between the first response data and the second response data (230). Here, the method may further comprise administering a sympatholytic drug after collecting the second response data (S430).

Specifically, first response data before perturbation during the autonomic nerve assessment test may be collected (S410). In addition, second response data after perturbation during the autonomic nerve assessment test may be collected (S420).

Next, the sympatholytic drug may be administered to the subject as described above (S430). The sympatholytic drug may be, for example, a vasodilator. In addition, the administration of such a sympatholytic drug may be performed using signals collected from an administration device or the like, or user input data. Accordingly, the autonomic nerve assessment test device may detect the above-mentioned collected signals or input data and recognize the administration of the sympatholytic drug.

Further, the third response data before perturbation during the autonomic nerve assessment test may be collected (S440).

The third response data may correspond to the first response data after administration of the sympatholytic drug. For example, the third response data may be the first response data before perturbation during the autonomic nerve assessment test after administering the sympatholytic drug to the patient. For example, after administering the sympatholytic drug, the third response data, which is response data in a supine or resting state, may be collected in a head-up tilt test.

In addition, it is possible to determine whether sympathectomy or sympathetic denervation is required using the first difference value between the first response data and the second response data and the second difference value, which is a difference value between the third response data and the second response data (S450 to S470). Specifically, it is possible to compare whether the ratio of the first difference value to the second difference value is within a predetermined range (S450). Further, if the ratio of the first difference value to the second difference value is within a predetermined range, it may be determined that sympathectomy or sympathetic denervation is required (S460). In contrast, if the ratio of the first difference value to the second difference value is greater than a predetermined range, it may be judged that sympathectomy or sympathetic denervation is not required (S470).

The method and device for predicting a subject for sympathectomy according to the present invention may utilize the fact that patients with pathologically high sympathetic nerve activity respond positively to some sympathetic nerve activity modulation therapies (e.g., renal denervation). The above-mentioned patients may respond differently in bioelectrical and hemodynamic signals when the sympathetic nerve or tone is disturbed than hypertensive patients whose sympathetic nerve or tone is not pathologically elevated. Accordingly, the method and device for predicting a subject for sympathectomy according to an embodiment may reflect the above facts to predict or judge whether a patient is a subject for sympathectomy. In other words, the above-mentioned test or examination may cause changes in sympathetic nerve activity. Further, the responses of bioelectrical and hemodynamic signals may be collected as response data.

For example, the hemodynamic and neurohormonal responses during the head-up tilt test may be considered part of a reflex response triggered by sympathetically induced hypercontraction of the nearly empty left ventricle. These hemodynamic and neurohormonal responses may appear qualitatively different in patients with and without abnormally high sympathetic activation (two groups). In particular, the peripheral blood pooling patterns of the two groups described above may be different. For example, other cardiovascular parameters such as central BP and left ventricular end-diastolic diameter (LVEDD) and stroke volume, which can be measured on echocardiography and/or bioimpedance signals, heart rate variability parameters on ECG, may be affected differently for the two groups. For example, plasma levels of epinephrine and norepinephrine may increase during the head-up tilt test. In this case, epinephrine and norepinephrine changes may be different between the two groups.

In addition, when the first response data or second response data is any one of central blood pressure, amount of epinephrine, and skin sympathetic nerve activity, the first response data may be lower than the second response data. Also, when the first response data or second response data is electrical resistance, the first response data may be higher than the second response data. When the first response data or second response data is heart rate variability, the first response data may be higher than the second response data in a low frequency region.

Accordingly, the method and device for predicting a subject for sympathectomy according to the present invention may particularly preferably utilize changes in some time series and/or static parameters from the response data (e.g., central blood pressure) measured non-invasively or invasively..

More specifically, exemplary parameters may include a difference value between central blood pressure (BP) values measured in supine position and head tilted position at various angles, a ratio between these differences, or combinations of these differences in temporary changes or in central blood pressure (BP) from the supine position to the head. In addition, the head-up tilt test may be performed regardless of whether medication is injected (e.g., administration of a sympatholytic drug). The methods disclosed herein may be implemented in several independent stand-alone monitors and may also be implemented in a production device for use in any therapy targeting patients with abnormally elevated sympathetic activity.

In addition, in an embodiment, in the case of a subject requiring sympathectomy, the second difference value may be greater than the first difference value. This is because in patients with abnormally high sympathetic nerve activation, sympathetic nerve activation is forcibly normalized by nitroglycerin. Therefore, the method and device for predicting a subject for sympathectomy according to an embodiment may more easily judge whether a sympathectomy is required.

Referring to FIG. 4, FIG. 4 shows first to third response data expressed as SSNA. First, (a) shows the third response data K3 of the general public or a patient. Also, (b) shows the first response data K1 of the general public. In addition, (c) shows the patient's first response data K1. Also, (d) shows the second response data K2 of the general public or patient. Also, as mentioned above, the general public may have a larger dose than the patient (RS1>RS2). Accordingly, for the general public and patients who are administered a sympatholytic drug and are in a resting state (before perturbation during the autonomic nerve assessment test), the response data, which is an indicator of the sympathetic nerve, represents the highest or lowest baseline value. As described above, the response data is data representing any one of central blood pressure, electrical resistance (bio impedance), heart rate variability (HRV), amount of epinephrine (or norepinephrine) in the blood, and skin sympathetic nerve activity (SSNA). Accordingly, the response data may decrease or increase from before the perturbation to after perturbation during the autonomic nerve assessment test, depending on the type of the response data. For example, in the case of SSNA, the response data increases in value from before perturbation to after perturbation during the autonomic nerve assessment test. That is, the first response data is smaller than the second response data. For example, K1 may be 40, K1' may be 20, K3 may be 10, and K2 may be 60 (unit is spike per 100 heartbeat).

Furthermore, in the present invention, the second difference value, which is the difference value between the third response data and the second response data, corresponds to 'GP1'. Also, the first difference value, which is the difference value between the first response data and the second response data, corresponds to 'RS2'. Further, in the present invention, the method judges whether sympathectomy is required by comparing the second difference value GP1 and the first difference value RS1. For example, in the case of SSNA, the second difference value GP1 may be greater than the first difference value RS2. However, it should be understood that this may not be the case when the first response data is greater than the second response data.

Referring to FIG. 5, the method for predicting a subject for sympathectomy according to another embodiment may comprise the steps of administering a sympatholytic drug (S510), collecting third response data before perturbation during an autonomic nerve assessment test (S520), collecting first response data before the perturbation during the nerve assessment test (S530), collecting second response data after the perturbation during the autonomic nerve assessment test (S540), and judging whether sympathectomy is required by using a first difference value between the first response data and the second response data (S550 to S570).

Specifically, a sympatholytic drug may be administered to the patient (S510), and then third response data before perturbation during the autonomic nerve assessment test may be collected (S520). The third response data is the first response data after administration of the sympatholytic drug. For example, the third response data may be the first response data after administering the sympatholytic drug to the patient and before the perturbation during the autonomic nerve assessment test. For example, after administering a sympatholytic drug, the third response data, which is response data in a supine or resting state, may be collected in a head-up tilt test. However, as described above, the first difference value and second difference value may be calculated using the first response data, second response data, and third response data.

In addition, the first response data before the perturbation during the autonomic nerve assessment test may be collected (S530). In this case, the first response data may be collected after the effect of administration of the sympatholytic drug is eliminated. That is, the first response data may be collected a predetermined time after the third response data is collected. Further, the second response data after the perturbation during the autonomic nerve assessment test may be collected (S540).

Further, it may be judged whether sympathectomy or sympathetic denervation is required using the first difference value and second difference value between the first response data and the second response data (S550 to S570). Specifically, it may compare whether a ratio of the first difference value to the second difference value is within a predetermined range (S550). The second difference value may be the difference between the second response data (calculated in S540) and the third response data (calculated in S520).

Further, if the ratio of the first difference value to the second difference value is within a predetermined range, it may be judged that sympathectomy or sympathetic denervation is required (S570). In contrast, when the ratio of the first difference value to the second difference value is greater than a predetermined range, it may be judged that sympathectomy or sympathetic denervation is not required (S560).

As such, in the method for predicting a subject for sympathectomy according to another embodiment, the collection of the first response data and second response data may be performed after the collection of the third response data. This may be applied differently depending on the patient's condition, etc.

FIG. 6 is a block diagram illustrating an example of a method and device for predicting a subject for sympathectomy according to an embodiment of the present invention, FIGS. 7 to 12 are graphs illustrating any one result of central blood pressure, electrical resistance (bio impedance), heart rate variability (HRV), amount of epinephrine in the blood, and skin sympathetic nerve activity (SSNA) for any one of head-up tilt test (HUT), Valsalva maneuver, and cold pressure test in an embodiment. FIG. 7 is a graph of central blood pressure, such as aortic systolic pressure, for a head-up tilt test, FIG. 8 is a graph of systemic vascular resistance index (SVRI) for a head-up tilt test, FIG. 9 is a graph of heart rate variability (power spectrum) for a head-up tilt test, FIG. 10 is a graph of epinephrine and norepinephrine in the blood for a head-up tilt test, FIG. 11 is a graph of skin sympathetic nerve activity for Valsalva maneuver, FIG. 12 is a graph of skin sympathetic nerve activity for a cold pressure test.

Referring to FIG. 6, in the method for predicting a subject for sympathectomy according to an embodiment, the head-up tilt test (HUT) during a test or examination may be performed by a change in state from a supine or rest/resting state to a tilt or incline state. Further, the device for predicting a subject for sympathectomy 100 according to an embodiment may receive the collected data and data on the state of the head-up tilt test from the patient and equipment for the head-up tilt test. Further, using the received data, it may determine whether it is a subject for sympathectomy (judge whether sympathectomy or sympathetic denervation is required) as described above.

Hereinafter, a means for collecting data (e.g., response data) for the above-mentioned test or examination (any one of central blood pressure, electrical resistance (bio impedance), heart rate variability (HRV), amount of epinephrine in the blood and skin sympathetic nerve activity (SSNA)) will be described by way of example.

Referring to FIG. 7, FIG. 7 illustrates a graph of central blood pressure, such as aortic systolic pressure, for a head-up tilt test. Likewise, sympathetic activity may cause vasoconstriction or vascular pressure. Accordingly, sympathetic nerve activity may be detected through changes in central blood pressure.

## Equation 1

$$DP_c = SBP_{supine} - SBP_{Tilt}$$

Here, $DP_c$ corresponds to the first difference value. $SBP_{supine}$ is response data in the supine or resting state in the head-up tilt test, and corresponds to the first response data. $SBP_{Tilt}$ is response data in a tilt state in the head-up tilt test and corresponds to the second response data.

## Equation 2

$$DP_n = SBPN_{supine} - SBP_{Tilt}$$

Here, $DP_n$ corresponds to the second difference value. $SBPN_{supine}$ is response data in the supine or resting state in the head-up tilt test after administration of a sympatholytic drug, and corresponds to the third response data.

In this case, this effect may be enhanced in the descending aorta as illustrated. Accordingly, when the central blood pressure is the aortic systolic pressure, a more accurate prediction of a subject for sympathectomy may be performed.

## Equation 3

$$SAP(Sympathetic\ Activity\ Reserve) = DP_c / DP_n$$

Here, SAP is the ratio of the first difference value to the second difference value.

In addition, in addition to the aortic systolic pressure described above, diastolic blood pressure, pulse pressure, aortic reflex time, enhancement index, etc., and combinations thereof may be applied to the response data. Furthermore, the response data and difference value calculation described above may be equally applied to the head-up tilt test, as well as the Valsalva maneuver and cold pressure test.

As illustrated, in patients with abnormally high sympathetic nerve activation, the change in response data (second and third response data) after administration of a sympatholytic drug (second difference value) and the change in response data after no administration of a sympatholytic drug (first difference value) may be different. The second difference value may be greater than the first difference value. As a result, the distinguishment for a patient with abnormally high sympathetic nerve activation, who is a subject for sympathectomy, may be accurately performed.

FIG. 8 is a graph of systemic vascular resistance index (SVRI) for a head-up tilt test. Referring to FIG. 8, sympathetic nerve activity may cause changes in body impedance, such as systemic vascular resistance coefficient. For example, when blood is pumped out strongly according to the sympathetic nerve, the impedance in the body may decrease, and if the blood is pumped more weakly, the impedance in the body may increase. Accordingly, sympathetic nerve activity may be detected through changes in systemic vascular resistance coefficient.

Furthermore, the same description may be applied not only to the systemic vascular resistance coefficient, but also to similar parameters (e.g., systemic vascular resistance index, heart rate, LVEDV reduction rate (left ventricular end-diastolic volume/or dimension), or a combination of these variables). This is explained in Equations 4 to 6 below.

## Equation 4

$$DZ_c = Z_{supine} - Z_{Tilt}$$

Here, $DZ_c$ corresponds to the first difference value. $SZ_{supine}$ is response data (systemic vascular resistance coefficient) in the supine or resting state in the head-up tilt test, and corresponds to the first response data. $Z_{Tilt}$ is the response data of the tilt state in the head-up tilt test and corresponds to the second response data (body vascular resistance coefficient).

## Equation 5

$$DZ_n = ZN_{supine} - Z_{Tilt}$$

Here, $DZ_n$ corresponds to the second difference value. $ZN_{supine}$ is response data in the supine or resting state in the head-up tilt test after administration of a sympatholytic drug, and corresponds to the third response data.

## Equation 6

$$SAP(Sympathetic\ Activity\ Reserve) = DZ_c\ /\ DZ_n$$

Here, SAP is the ratio of the first difference value to the second difference value.

In this way, various parameter values that may indicate sympathetic nerve activation may be applied to the response data. By way of example, the response data may be data about at least one of central blood pressure, electrical resistance (bio impedance), heart rate variability (HRV), amount of epinephrine in the blood, and skin sympathetic nerve activity (SSNA), as described above.

As shown, in the case of patients with abnormally high sympathetic nerve activation, as in FIG. 7, the change in response data after administration of a sympatholytic drug (second difference value) and the change in response data after non-administration of a sympatholytic drug (first difference value) may be different from each other. The second difference value may be greater than the first difference value. As a result, a patient with abnormally high sympathetic nerve activation, who is a subject for sympathectomy may be accurately distinguished.

FIG. 9 is a graph of heart rate variability (power spectrum) for a head-up tilt test. Referring to FIG. 9, when changing from a supine or resting state to a tilt or incline state during the head-up tilt test, heart rate variation may also be appear differently in a patient with abnormally high sympathetic nerve activation (b) and a patient without abnormal high sympathetic nerve activation (A, normal).

In the case of a patient (a) with normal sympathetic nerve activation, when changing from supine to tilt in the head-up tilt test, the level at low frequency LF, which represents the sympathetic nerve, may increase. Further, the size at high frequency HF, which represents the parasympathetic nervous system, may decrease.

On the other hand, in the case of a patient (b) with abnormally high sympathetic nerve activation, when changing from a lying state to a tilt in the head-up tilt test, the size at low frequency LF, which represents the sympathetic nerve, may not change significantly. For example, the sympathetic nerve may refer to a low frequency range of 0.04 to 0.15 Hz. Preferably it may be in the range of 0 to 0.2hz. In addition, parasympathetic nerves may refer to high frequency in the range of 0.15 to 0.4 Hz. Preferably, it may be in the range of 0.2Hz to 0.3Hz.

Accordingly, by using the spectrum of the illustrated electrocardiogram as the response data, it may perform accurate distinguishment for the patients with abnormally high sympathetic nerve activation, who are subject for sympathectomy.

For example, the electrocardiogram (ECG) of heart rate variability may apply parameters in both the time domain and the frequency domain. For example, the response data may include SDNN, SDANN, RMSS, pNN50, etc.

FIG. 10 is a graph of epinephrine and norepinephrine in the blood for a head-up tilt test. Referring to FIG. 10, a first group (group 1) is a normal patient, a second group (group2) is a normal patient with syncope during the head-up tilt test, and a third group (group3) is a patient with syncope of unknown cause.

In the head-up tilt test (from lying down to tilting), changes in epinephrine and norepinephrine in normal patients (group 1, group 2) may not be as large as those in abnormal patients (group 3). In this way, it may perform accurate distinguishment for the patients with abnormally high sympathetic nerve activation (group 3), who are subjects for sympathectomy.

FIG. 11 is a graph of skin sympathetic nerve activity for Valsalva maneuver, and FIG. 12 is a graph of skin sympathetic nerve activity for a cold pressure test.

Referring to FIG. 11, it may be seen that SKNA, iSKNA, HR, and ECG all increase as the Valsalva maneuver is applied. The second response data may be larger than the first response data. Likewise, in patients with abnormally high sympathetic nerve activation, the change in response data (second difference value) after administration of a sympatholytic drug may be different from the change in response data (first difference value) after non-administration of a sympatholytic drug. Further, the second difference value may be greater than the first difference value. As a result, the patient with abnormally high sympathetic nerve activation who is a subject for sympathectomy can be accurately distinguished.

Referring to FIG. 12, it may be seen that SKNA, iSKNA, and HR all increase when a cold pressure test (CPT) is performed, as described above. Accordingly, the second response data may be larger than the first response data. However, the patient with abnormally high sympathetic nerve activation (subject 4) showed no significant correlation with the increase in heart rate compared to normal patients (subject 1 to subject 3).

In this way, skin sympathetic nerve activity may be used to more accurately distinguish patients with abnormally high sympathetic nerve activation. Such distinguishment may not be detected on the basis of heart rate.

The method for predicting a subject for sympathectomy according to the disclosed embodiment may be implemented in the form of program instructions that may be executed through various computer means and recorded on a computer-readable medium. In addition, an embodiment of the present disclosure may be a computer-readable recording medium on which one or more programs including instructions for executing a method for predicting a subject for sympathectomy are recorded.

Further, the computer-readable medium may include a program instruction, a data file, a data structure, or a combination thereof. The program instructions recorded on the computer-readable medium may be designed and configured specifically for the present invention or may be publicly known and available to those who are skilled in the field of computer software. The computer-readable medium includes a magnetic media such as a hard disk, a floppy disk and a magnetic tape, an optical media such as a CD-ROM and a DVD, a magneto-optical media such as a floptical disk, and a hardware device, such as a ROM, a RAM, and a flash memory, which is specially configured to store and execute the program command. The example of the program instruction includes a high level language code, which can be executed by a computer through an interpreter, as well as a machine language code as made by a compiler.

Herein, the machine-readable storage media may be provided in the form of a non-transitory storage medium. In this case, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium. For example, a 'non-transitory storage medium' may include a buffer where data is temporarily stored.

According to an embodiment, a method for predicting a subject for sympathectomy according to various embodiments disclosed in this document may be provided while being included in a computer program product. The computer program products may be traded between a seller and a purchaser as merchandise. The computer program products may be distributed in the form of a device readable storage medium (e.g., compact disc read only memory (CD-ROM) or distributed (e.g., downloaded or uploaded) online directly through an application store (e.g., Play Store™) or between two user devices (e.g., smartphones). In the case of online distribution, some of the computer program products (e.g., downloadable app) may be at least transitorily stored in a device readable storage medium such as a server of a manufacturer, a server of the application store, or a memory of a relay server, or temporarily generated.

Specifically, the present invention may be implemented in the form of a computer program product including a recording medium having recorded thereon a program configured to perform the method for predicting a subject for sympathectomy according to the disclosed embodiment.

Although the embodiments have been described in detail above, the scope of the present invention is not limited thereto, and various modifications and improvements by those skilled in the art using the basic concept of the present invention defined in the following claims also belong to the scope of the present invention.

A term of 'unit' used in the embodiment means software or a hardware component such as a field-programmable gate array (FPGA) or ASIC, and 'unit' performs predetermined roles. However, 'unit' is not a meaning limited to software or hardware. 'Unit' may be configured to be positioned in an addressable storage medium and configured to regenerate one or more processors. Therefore, as one example, 'unit' includes components such as software components, object oriented software components, class components, and task components, processes, functions, attributes, procedures, subroutines, segments of a program code, drivers, firmware, a microcode, a circuit, data, a database, data structures, tables, arrays, and variables. Functions provided in the components and 'units' may be joined as a smaller number of components or further separated into additional components and 'units'. The components and 'units' may be implemented to regenerate one or more CPUs within a device or a security multimedia card.

While the present invention is described mainly based on the above embodiments but is not limited thereto, it will be understood by those skilled in the art that various changes and modifications are made without departing from the spirit and scope of the present invention. For example, each component specifically shown in the embodiments may be modified and implemented. It should be interpreted that differences relating to such modifications and application are included in the scope of the present invention defined in the appended claims.

**Claims**

1. A method for predicting a subject for sympathectomy, comprising the steps of:

    collecting first response data before a perturbation during an autonomic nerve assessment test;
    collecting second response data after the perturbation during the autonomic nerve assessment test; and
    judging whether a sympathectomy is required, by using a first difference value between the first response data and the second response data,
    wherein the autonomic nerve assessment test is any one of head-up tilt (HUT) test, Valsalva maneuver, and cold pressure test.

2. The method of claim 1, comprising the step of collecting third response data before the perturbation during the autonomic nerve assessment test,
    wherein the third response data is the first response data after administration of a sympatholytic drug.

3. The method of claim 2, wherein if a ratio of the first difference value to a second difference value is within a predetermined range, it is judged that the sympathectomy or sympathetic denervation is required,
    wherein the second difference value is a difference value between the second response data and the third response data.

4. The method of claim 2, wherein the second difference value is greater than the first difference value,
    the second difference value is a difference value between the second response data and the third response data.

5. The method of claim 1, wherein in the judgment step, it is judged that the sympathectomy or sympathetic denervation is required when a ratio of the first difference value to an average difference value is within a predetermined range.

6. The method of claim 1, wherein the first response data or the second response data is any one of central blood pressure, electrical resistance (bio impedance), heart rate variability (HRV), amount of epinephrine/norepinephrine in blood and skin sympathetic nerve activity (SSNA).

7. A non-transitory computer-readable storage medium on which a program including at least one instruction for performing a method for predicting a subject for sympathectomy is recorded, wherein the method for predicting a subject for sympathectomy comprising the steps of:

    collecting first response data before a perturbation during an autonomic nerve assessment test;
    collecting second response data after the perturbation during the autonomic nerve assessment test; and
    judging whether a sympathectomy is required, by using a first difference value between the first response data and the second response data,
    wherein the autonomic nerve assessment test is any one of head-up tilt (HUT) test, Valsalva maneuver, and cold pressure test.

8. A device for predicting a subject for sympathectomy comprising a processor performing at least one instruction, wherein the processor is configured to:

    collect first response data before a perturbation during an autonomic nerve assessment test;
    collect second response data after the perturbation during the autonomic nerve assessment test; and
    judge whether a sympathectomy is required, by using a first difference value between the first response data and the second response data,

wherein the autonomic nerve assessment test is any one of head-up tilt (HUT) test, Valsalva maneuver, and cold pressure test.

9. The device of claim 8, wherein the processor is configured to collect third response data before the perturbation during the autonomic nerve assessment test,

   wherein the third response data is the first response data after administration of a sympatholytic drug.

10. The device of claim 9, wherein the processor is configured to judge that the sympathectomy or sympathetic denervation is required if a ratio of the first difference value to a second difference value is within a predetermined range,

    wherein the second difference value is a difference value between the second response data and the third response data.

100

140

Communication
Unit

110

First Sensor
Unit

130

Input Unit

Processor ～160

Second Sensor
Unit

Output Unit

120

150

Storage Unit

170

FIG. 1

```
                        ┌─────────────┐
                        │    start    │
                        └─────────────┘
                               │
                               ▼
        ┌────────────────────────────────────────────────┐
        │ collect first response data before perturbation during autonomic │  ~S210
        │            nerve assessment test               │
        └────────────────────────────────────────────────┘
                               │
                               ▼
        ┌────────────────────────────────────────────────┐
        │ collect second response data after perturbation during autonomic │  ~S220
        │            nerve assessment test               │
        └────────────────────────────────────────────────┘
                               │
                               ▼
        ┌────────────────────────────────────────────────┐
        │judge whether sympathectomy is required, by using a first difference │  ~S230
        │value between the first response data and the second response data│
        └────────────────────────────────────────────────┘
                               │
                               ▼
                        ┌─────────────┐
                        │     end     │
                        └─────────────┘
```

## FIG. 2A

(a)

rs1

Supine
resting

HUT
V.M
C.P.

(b)

rs2

Supine
resting

HUT
V.M
C.P.

## FIG. 2B

FIG. 3

FIG. 4

FIG. 5

EP 4 527 288 A1

FIG. 6

FIG. 7

FIG. 8

FIG. 9

FIG. 10

FIG. 11

FIG. 12

## INTERNATIONAL SEARCH REPORT

| International application No. |
| --- |
| **PCT/KR2023/006669** |

**A.    CLASSIFICATION OF SUBJECT MATTER**

**A61B 5/00**(2006.01)i; **A61B 5/021**(2006.01)i; **A61B 5/053**(2006.01)i; **A61B 5/024**(2006.01)i; **A61B 5/145**(2006.01)i

According to International Patent Classification (IPC) or to both national classification and IPC

**B.    FIELDS SEARCHED**

Minimum documentation searched (classification system followed by classification symbols)

A61B 5/00(2006.01); A61B 18/00(2006.01); A61B 18/12(2006.01); A61B 18/14(2006.01); A61B 5/021(2006.01); A61B 5/04(2006.01); A61B 5/0476(2006.01); A61B 5/055(2006.01); A61N 7/00(2006.01); G09F 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 자율신경(autonomic nerve), 동요(perturbation), 교감신경(sympathetic nerve), 평가(evaluation), 절제(ablation), 예측(estimate), 기립경사검사(HUT), 발살바수기(valsalva maneuver), 한랭압박검사(cold pressure test)

**C.    DOCUMENTS CONSIDERED TO BE RELEVANT**

| Category* | Citation of document, with indication, where appropriate, of the relevant passages | Relevant to claim No. |
| --- | --- | --- |
| Y | KR 10-2010-0038107 A (NEUROFOCUS, INC.) 12 April 2010 (2010-04-12)<br>See claims 1-9. | 1-10 |
| Y | US 9820811 B2 (SYMAP HOLDING LIMITED) 21 November 2017 (2017-11-21)<br>See claim 1. | 1-10 |
| Y | KR 10-2018-0116185 A (MEDICORE CO., LTD.) 24 October 2018 (2018-10-24)<br>See paragraph [0194]. | 1-10 |
| A | WO 2016-054379 A1 (MEDTRONIC ARDIAN LUXEMBOURG S.A.R.L.) 07 April 2016 (2016-04-07)<br>See entire document. | 1-10 |
| A | US 2014-0336497 A1 (KONA MEDICAL, INC.) 13 November 2014 (2014-11-13)<br>See entire document. | 1-10 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **22 August 2023** | **22 August 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/006669**

| Patent document cited in search report | | | Publication date (day/month/year) | Patent family member(s) | | | Publication date (day/month/year) |
|---|---|---|---|---|---|---|---|
| KR | 10-2010-0038107 | A | 12 April 2010 | CN | 101815467 | A | 25 August 2010 |
| | | | | CN | 101815467 | B | 17 July 2013 |
| | | | | EP | 2170161 | A1 | 07 April 2010 |
| | | | | EP | 2170161 | A4 | 09 January 2013 |
| | | | | EP | 2170161 | B1 | 05 December 2018 |
| | | | | JP | 2010-535083 | A | 18 November 2010 |
| | | | | JP | 5542051 | B2 | 09 July 2014 |
| | | | | US | 10733625 | B2 | 04 August 2020 |
| | | | | US | 11244345 | B2 | 08 February 2022 |
| | | | | US | 2009-0036756 | A1 | 05 February 2009 |
| | | | | US | 2013-0332259 | A1 | 12 December 2013 |
| | | | | US | 2019-0139078 | A1 | 09 May 2019 |
| | | | | US | 2020-0364741 | A1 | 19 November 2020 |
| | | | | US | 2022-0230199 | A1 | 21 July 2022 |
| | | | | US | 8533042 | B2 | 10 September 2013 |
| | | | | WO | 2009-018374 | A1 | 05 February 2009 |
| US | 9820811 | B2 | 21 November 2017 | US | 10722303 | B2 | 28 July 2020 |
| | | | | US | 11576721 | B2 | 14 February 2023 |
| | | | | US | 2016-0081744 | A1 | 24 March 2016 |
| | | | | US | 2018-0078307 | A1 | 22 March 2018 |
| | | | | US | 2020-0383725 | A1 | 10 December 2020 |
| KR | 10-2018-0116185 | A | 24 October 2018 | CN | 105960643 | A | 21 September 2016 |
| | | | | KR | 10-2015-0110342 | A | 02 October 2015 |
| | | | | KR | 10-2018-0116184 | A | 24 October 2018 |
| | | | | KR | 10-2122231 | B1 | 15 June 2020 |
| | | | | KR | 10-2122240 | B1 | 16 June 2020 |
| WO | 2016-054379 | A1 | 07 April 2016 | EP | 3200712 | A1 | 09 August 2017 |
| | | | | EP | 3200712 | B1 | 25 November 2020 |
| | | | | EP | 3791817 | A1 | 17 March 2021 |
| | | | | US | 10368775 | B2 | 06 August 2019 |
| | | | | US | 11311205 | B2 | 26 April 2022 |
| | | | | US | 2016-0095535 | A1 | 07 April 2016 |
| | | | | US | 2019-0307361 | A1 | 10 October 2019 |
| | | | | US | 2022-0240807 | A1 | 04 August 2022 |
| US | 2014-0336497 | A1 | 13 November 2014 | US | 2012-0065492 | A1 | 15 March 2012 |
| | | | | US | 2013-0253381 | A1 | 26 September 2013 |
| | | | | US | 2014-0194784 | A1 | 10 July 2014 |
| | | | | US | 2014-0194785 | A1 | 10 July 2014 |
| | | | | US | 8517962 | B2 | 27 August 2013 |

Form PCT/ISA/210 (patent family annex) (July 2022)